# EUROPEAN PATENT APPLICATION

(11) **EP 4 585 132 A1**
(43) Date of publication of application: **16.07.2025**
(21) Application number: 23911283.2
(22) Date of filing: 11.09.2023
(51) Int. Cl.: A61B 1/00, G02B 23/24

(54) **ENDOSCOPE HOOD**

(30) Priority: 27.12.2022 JP 2022210531
(71) Applicant: Seed Co., Ltd., Tokyo 113-8402 (JP)
(72) Inventor: SATO Takao, Tokyo 113-8402 (JP); MATSUNAGA Toru, Tokyo 113-8402 (JP); KANEDA Shiori, Tokyo 113-8402 (JP); TAKASU Yuka, Tokyo 113-8402 (JP); MORITA Yoshinori, Kobe-shi, Hyogo 657-8501 (JP); SHIMAMOTO Yusaku, Kobe-shi, Hyogo 657-8501 (JP)
(74) Representative: Viering, Jentschura & Partner mbB Patent- und Rechtsanwälte
(86) International application number: PCT/JP2023/033063
(87) International publication number: WO 2024/142499

(57) **Abstract**

The object of the present invention is to provide an endoscope hood having, in addition to antifogging properties and antifouling properties, in particular, strength and elastic force sufficient for a distal end portion to push apart a biological tissue. The endoscope hood (1) includes: a main body portion (5) made of a hydrogel; and a second distal end portion (7), wherein the main body portion (5) includes: an exterior portion (2) having a substantially cylindrical shape, a disk portion (3) circumferentially in contact with an inner surface of the exterior portion (2), and a first distal end portion (4) connected to an insertion-side end portion of the exterior portion (2), and the second distal end portion (7) is connected to an insertion-side end portion of the first distal end portion(4) and includes an annular member.

## Description

### TECHNICAL FIELD

The present invention relates to an endoscope hood.

### BACKGROUND ART

In the endoscope, a plurality of through-holes are formed to penetrate in the longitudinal direction, and a camera lens and an illumination lens for ensuring a field of view are arranged in the through-holes.

During the surgery or examination using an endoscope, a transparent endoscope hood made of ABS, polycarbonate, vinyl chloride, or the like is attached to the distal end portion of the endoscope for the purpose of confirming an optimal distance from the distal end portion of the endoscope to a biological tissue and ensuring a field of view. Furthermore, in recent years, a transparent endoscope hood made of silicone rubber or the like is attached to the distal end portion of the endoscope for the purpose of further preventing damage to biological tissue.

However, such an endoscope hood is attached to a distal end portion of an endoscope for the purpose of confirming an optimal distance from the distal end portion of the endoscope to a biological tissue and ensuring a field of view, but a part of the camera lens or illumination lens is not covered. Thus, there is a problem that body fluids or oil may adhere and the field of view may become unclear during examinations or surgeries using the endoscope to which such an endoscope hood is attached.

Therefore, for the purpose of improving contamination resistance of the camera lens and illumination lens, an endoscope hood in which the lens part can be covered is formed and further subjected to antifogging treatment (refer to Patent Literature 1 and Patent Literature 2), an endoscope hood provided with a hydrophilic coating (refer to Patent Literature 3 and Patent Literature 4), and the like are known.

Furthermore, a technique of incorporating a liquid spraying mechanism into an endoscope and cleaning a lens part (refer to Patent Literature 5) is also known.

### CITATION LIST

### PATENT LITERATURE

Patent Literature 1: Japanese Patent Application Laid-Open No. H11-047081 (JP-A H11-047081)
Patent Literature 2: Japanese Patent Application Laid-Open No. 2004-267583 (JP-A 2004-267583)
Patent Literature 3: Japanese Patent Application Laid-Open No. 2007-151685 (JP-A 2007-151685)
Patent Literature 4: Japanese Patent Application Laid-Open No. 2009-213631 (JP-A 2004-213631)
Patent Literature 5: Japanese Patent Application Laid-Open No. 2009-240596 (JP-A 2009-240596)

### SUMMARY OF THE INVENTION

### TECHNICAL PROBLEM

However, since the endoscope hood and the lens are formed of a material having high lipophilicity, affinity with body fluids and oil is extremely high, and the adhered body fluids and oil cannot be completely removed in long-term use, and the antifogging and antifouling technologies described in Patent Literature 1 to 5 cannot be said to have sufficient effects.

In addition, in a case where there is significant fogging or contamination, it is necessary to temporarily suspend surgery or examination, remove the endoscope, clean the contamination, and then reinsert the endoscope. This leads to a problem of a long operation time and a burden on the patient due to reinsertion. Therefore, there has been a demand for development of an endoscope hood excellent in contamination resistance.

Therefore, the present inventors have developed an endoscope hood having excellent antifogging properties and antifouling properties by forming the entire endoscope hood with a hydrogel.

Meanwhile, since the vicinity of the distal end of the endoscope hood also has a function of pushing apart the biological tissue, flexibility and strength to the extent that the biological tissue is not damaged are required in addition to the antifogging properties and the antifouling properties.

In addition, the inventor of the present application has confirmed that the antifogging properties and the antifouling properties are remarkably improved as the entire endoscope hood exhibits excellent hydrophilicity simply by forming the endoscope hood from the hydrogel. However, the high flexibility peculiar to the hydrogel cannot be said to be optimal for pushing apart the biological tissue, and there is room for improvement in the function for pushing apart the biological tissue, particularly in the distal end portion of the endoscope hood.

Therefore, the present invention has been made to solve the above-described problems, and an object of the present invention is to provide an endoscope hood having, in addition to antifogging properties and antifouling properties, in particular, strength and elastic force sufficient for a distal end portion to push apart a biological tissue.

### SOLUTION TO PROBLEM

An endoscope hood according to an embodiment is summarized as including: a main body portion made of a hydrogel; and a second distal end portion, wherein the main body portion includes: an exterior portion having a substantially cylindrical shape, a disk portion circumferentially in contact with an inner surface of the exterior portion, and a first distal end portion connected to an insertion-side end portion of the exterior portion, and the second distal end portion is connected to an insertion-side end portion of the first distal end portion and includes an annular member.

### ADVANTAGEOUS EFFECTS OF INVENTION

According to the present invention, it is possible to provide an endoscope hood having, in addition to antifogging properties and antifouling properties, in particular, strength and elastic force sufficient for a distal end portion to push apart a biological tissue.

### BRIEF DESCRIPTION OF THE DRAWINGS

[FIG. 1] Fig. 1 is an example of a cross-sectional view and a plan view of an endoscope hood 1 according to an embodiment.
[FIG. 2] Fig. 2 is a diagram illustrating an example of a state where the endoscope hood according to the embodiment is attached.

### DESCRIPTION OF EMBODIMENTS

Hereinafter, embodiments will be described in detail with reference to the drawings as appropriate. However, unnecessarily detailed description will be omitted in some cases. For example, a detailed description of a well-known matter and a repeated description of substantially the same configuration will be omitted in some cases. This is to avoid unnecessary redundancy of the following description and to facilitate understanding of those skilled in the art. Note that the inventors provide the accompanying drawings and the following description in order for those skilled in the art to fully understand the present disclosure, and does not intend to limit the subject matter described in the claims by the accompanying drawings and the following description.

In the following description of the drawings, the same or similar parts will be given the same or similar reference numerals. However, it should be noted that the drawings are schematic, and ratios of dimensions and the like may be different from actual ones. Therefore, specific dimensions and the like should be determined in consideration of the following description. In addition, the drawings may include parts having different dimensional relationships and ratios.

### <First Embodiment>

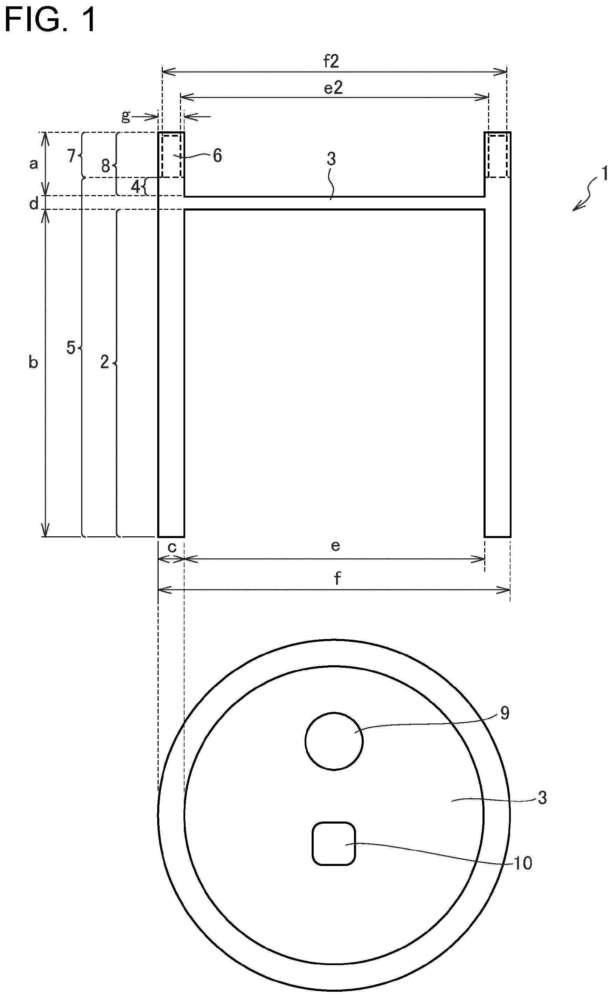

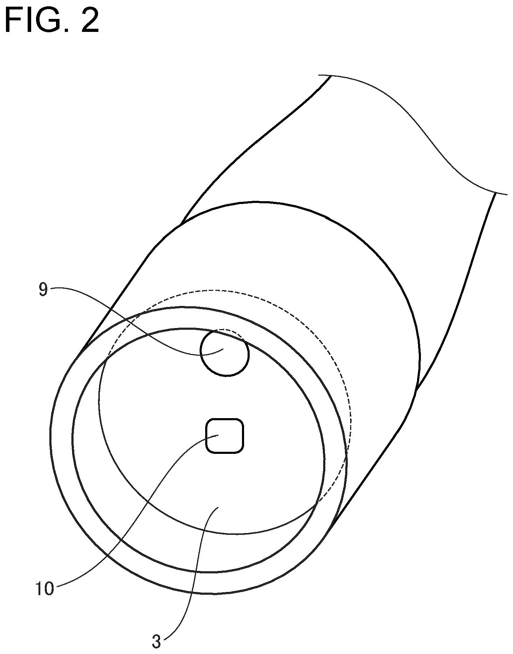

An endoscope hood 1 according to a first embodiment of the present invention will be described with reference to Figs. 1 and 2. Fig. 1 is an example of a cross-sectional view and a plan view of the endoscope hood 1 according to the present embodiment, and Fig. 2 is a view illustrating an example of a state where the endoscope hood according to the embodiment is attached.

The endoscope hood 1 according to the present embodiment is an endoscope hood made of a hydrogel, and includes a main body portion 5 and a second distal end portion 7 as illustrated in Fig. 1.

As illustrated in Fig. 1, the main body portion 5 includes an exterior portion 2, a disk portion 3, and a first distal end portion 4.

The exterior portion 2 has a substantially cylindrical shape, and is configured to be in contact with the outer surface of the endoscope at the inner surface to fix the endoscope hood 1 to the endoscope.

The length b of the exterior portion 2 is not particularly limited as long as the length b is a length sufficient to fix the endoscope hood 1 to the endoscope, and is preferably 5 to 20 mm, and more preferably 10 to 15 mm. The thickness c of the exterior portion 2 is preferably 0.1 to 1.0 mm, and more preferably 0.3 to 0.5 mm. Furthermore, the inner diameter e of the exterior portion 2 can be appropriately designed according to the endoscope to be used.

The disk portion 3 has a disk shape, and is provided in circumferential contact with the inner surface of the exterior portion 2. The disk portion 3 covers the insertion-side end portion of the endoscope except for having a forceps port 9 serving as the inlet and outlet of the forceps and a water supply port 10 serving as the outlet of the cleaning water, and is configured to prevent the camera lens from being contaminated.

The disk portion 3 is formed in a substantially flat surface, and the thickness d of the disk portion 3 is preferably 0.01 to 1.0 mm, more preferably 0.05 to 0.7 mm, and most preferably 0.1 to 0.5 mm. When the thickness d of the disk portion 3 exceeds 1.0 mm, the field of view obtained through the camera lens is adversely affected. In addition, when the thickness d of the disk portion 3 is smaller than 0.01 mm, the strength of the disk portion 3 becomes low, and there is a concern that breakage may occur during use, which is not preferable.

The first distal end portion 4 has a cylindrical shape formed with substantially the same dimension as the inner diameter e and the outer diameter f of the exterior portion 2, is provided to be connected to the insertion-side end portion of the exterior portion 2, and is configured to form a part of the distal end portion 8.

In addition, the second distal end portion 7 also has a cylindrical shape formed with substantially the same dimensions as the inner diameter e and the outer diameter f of the exterior portion 2, and is provided to be connected to the insertion-side end portion of the first distal end portion 4. Here, the second distal end portion 7 includes an annular portion 6.

The length a of the distal end portion 8 formed by the first distal end portion 4 and the second distal end portion 7 is not particularly limited as long as an optimal distance and a field of view can be ensured, and is preferably 1 to 10 mm, and more preferably 2 to 5 mm. Here, when the length a of the distal end portion 8 exceeds 10 mm, the reflection of the distal end portion on the camera lens increases, and the field of view is narrowed, which is not preferable.

The thickness g of the distal end portion 8 is preferably 0.1 to 1.0 mm, and more preferably 0.3 to 0.5 mm.

The 30% deformation compressive bending strength of the annular portion 6 is preferably stronger than the 30% deformation compressive bending strength of the main body portion 5.

According to such a configuration, by improving the strength of the distal end portion 8 (second distal end portion 7) including the annular portion 6 as compared with the strength of the main body portion 5, it is possible to impart appropriate strength for pushing apart the biological tissue to the distal end portion 8 of the hydrogel endoscope hood 1 according to the present embodiment.

The 30% deformation compressive bending strength of the annular portion 6 is preferably 1.0 N to 2.0 N.

When the 30% deformation compressive bending strength of the annular portion 6 is less than 1.0 N, the function for pushing apart the biological tissue is not imparted to the distal end portion 8 of the hydrogel endoscope hood 1 according to the present embodiment, and when the 30% deformation compressive bending strength of the annular portion 6 exceeds **2.0 N,** there is a risk of damaging the distal end portion of the main body when the hydrogel endoscope hood 1 according to the present embodiment is used, which is not preferable.

As the method for forming the second distal end portion 7 according to the present embodiment, a first aspect can be adopted in which the annular member 6 formed in advance is disposed at a predetermined position of the molding die for forming the endoscope hood 1, and then the polymerizable composition for forming the main body portion 5 is injected and subjected to a copolymerization reaction.

With respect to the shape of the annular member 6 in the first aspect, by forming the inner diameter e2 to be larger than the inner diameter e of the exterior portion 2 and forming the outer diameter f2 to be smaller than the outer diameter f of the exterior portion 2, the annular member 6 is enclosed in the endoscope hood 1 in the step of injecting the polymerizable composition for forming the main body portion 5 and subjecting the polymerizable composition to a copolymerization reaction.

Such a configuration is preferable because contamination resistance is imparted to the distal end portion 8 (second distal end portion 7) including the annular member 6.

In the endoscope hood 1 forming step according to the present embodiment, pretreatment such as plasma irradiation or corona discharge is performed before the annular member 6 is placed on the jig for forming the main body portion 5, whereby the affinity between the annular member 6 and the body portion 5 can be improved in the copolymerization reaction.

As the method for forming the second distal end portion 7 according to the present embodiment, a second aspect can be adopted in which a predetermined amount of the polymerizable composition is injected into the end portion on the insertion side of the molding die for forming the endoscope hood 1, the annular member 6 is formed by being subjected to a polymerization reaction, and then the polymerizable composition for forming the main body portion 5 is continuously injected, and the mixture is subjected to a further copolymerization reaction.

In the second aspect, the inner diameter e2 and the outer diameter f2 of the annular member 6 have the same values as the inner diameter e and the outer diameter f of the exterior portion 2, respectively.

In the first aspect, examples of the material forming the annular member 6 of the endoscope hood 1 according to the present embodiment include thermoplastic elastomers selected from polystyrene-based, olefin-based, PVC-based, polyurethane-based, polyester-based, polyamide-based, EVA-based, acryl-based, and the like, and thermosetting elastomers selected from vulcanized rubber, resin-based elastomers, and the like.

In the second aspect, examples of the material forming the annular member 6 of the endoscope hood 1 according to the present embodiment include a crosslinked hydrophilic polymer (hydrogel).

As a method for molding the annular member 6, the annular member 6 can be processed into a desired shape by appropriately selecting a molding method suitable for the material to be used from known methods such as injection molding, cast molding, and cutting molding.

When a crosslinked hydrophilic polymer (hydrogel) is selected as the material of the annular member 6, the polymerizable composition for forming the annular member 6 may have a composition that is the same as or different from the polymerizable composition for forming the main body portion 5 to be described later. In particular, in the same case, it is important that the amount of the crosslinkable monomer to be blended in the polymerizable composition for forming the annular member 6 is larger than the amount of the crosslinkable monomer to be blended in the polymerizable composition for forming the main body portion 5.

Although the specific blending amount will be described later, as described above, by increasing the amount of the crosslinkable monomer, the crosslinking density of the annular member 6 becomes higher than the crosslinking density of the main body portion 5, and the strength is improved. Therefore, when the annular member 6 (second distal end portion 7) and the main body portion 5 are combined to form the endoscope hood 1 according to the present embodiment, it is possible to impart preferable strength to the distal end portion 8.

Although the strength of the annular member 6 in the present invention is described in detail in Examples, in the present invention, evaluation is performed by 30% deformation compressive bending strength (N) when the annular member 6 is loaded, and the preferable 30% deformation compressive bending strength is 1.0 to 2.0 (N), more preferably 1.1 to 1.9 (N), and most preferably 1.2 to 1.8 (N).

When the 30% deformation compressive bending strength of the annular portion 6 is less than 1.0 (N), the strength is not sufficient to push apart the biological tissue, and when the 30% deformation compressive bending strength of the annular portion 6 exceeds 2.0 N, there is a risk that the distal end portion of the main body may be damaged during the use of the hydrogel endoscope hood 1 according to the present embodiment, which is not preferable.

In the endoscope hood 1 according to the present embodiment, the main body portion 5 is made of a hydrogel. Examples of the hydrogel include a hydrogel formed using only a hydrophilic monomer, and a hydrogel formed by adding a hydrophobic monomer, a crosslinkable monomer, or both to a hydrophilic monomer.

The hydrophilic monomer contributes to the water content of the hydrogel, and the hydrophobic monomer contributes to the action of adjusting the water content and swelling rate of the hydrogel, thereby affecting the wettability and flexibility of the endoscope hood 1 to be obtained.

In addition, the crosslinkable monomer can control the density of polymer chains of the hydrogel depending on the content thereof, and can impart mechanical strength, shape stability, and solvent resistance to the hydrogel.

The water content (moisture content (wt%) = [(W - D)/W] × 100 (W: water-containing weight, D: dry weight)) of the hydrogel is not particularly limited, and can be, for example, 20 to 70 wt%. In addition, the water content can be appropriately selected as necessary.

As the hydrophilic monomer, those having one or more hydrophilic groups in the molecule are preferable, and examples thereof include 2-hydroxyethyl (meth)acrylate, 2-hydroxymethyl (meth)acrylate, hydroxypropyl (meth)acrylate, glycerol (meth)acrylate, acrylamide, N,N-dimethyl (meth)acrylamide, N,N-diethyl (meth)acrylamide, N-vinylpyrrolidone, diacetone acrylamide, N-vinylacetamide, (meth)acrylic acid, (meth)acryloxyethyl succinic acid, itaconic acid, methacrylamidopropyl triammonium chloride, 2,3-dihydroxypropyl (meth)acrylate, and the like, and at least one or a combination of two or more thereof may be used.

Among the hydrophilic monomers described above, 2-hydroxyethyl (meth)acrylate, N,N-dimethyl (meth)acrylamide, and N-vinylpyrrolidone are preferably used in the present invention from the viewpoint of handleability.

The blending ratio of the hydrophilic monomer is not particularly limited, but is preferably 50 wt% or more in all the polymerized components since it affects the water content of the endoscope hood 1 to be obtained. When the blending ratio of the hydrophilic monomer is less than 50 wt%, the endoscope hood 1 having a sufficient water content cannot be obtained, and thus there is a concern that the antifouling properties and the antifogging properties of the endoscope hood 1 may be deteriorated, which is not preferable.

Examples of the hydrophobic monomer include siloxanyl (meth)acrylate, trifluoroethyl (meth)acrylate, methacrylamide, cyclohexyl (meth)acrylate, and normal butyl (meth)acrylate, and two or more of these hydrophobic monomers may be used in combination.

The hydrophobic monomer can change the water content of the endoscope hood 1 obtained according to the blending amount. However, when the blending ratio of the hydrophobic monomer is high, the water content is extremely lowered, and the flexibility of the endoscope hood 1 to be obtained is lowered. Therefore, for example, the blending ratio of the hydrophobic monomer is preferably less than 30 wt% with respect to the total amount of monomers.

Examples of the crosslinkable monomer include ethylene glycol di(meth)acrylate, polyethylene glycol di(meth)acrylate, methylene bisacrylamide, 2-hydroxy-1,3-dimethacryloxypropane, and trimethylolpropane triacrylate, and at least one of these monomers or a combination of two or more thereof may be used.

The blending amount of the crosslinkable monomer is preferably 0.1 to 10 wt% with respect to the total amount of monomers from the viewpoint of the effect of adjusting the shape of the endoscope hood 1 to be obtained. When the blending amount is less than 0.1 wt%, the network structure of the endoscope hood 1 is insufficient, and when the content exceeds 10 wt%, the network structure becomes excessive, and thus the endoscope hood 1 becomes brittle and the flexibility decreases.

Examples of the polymerization initiator used for polymerizing the mixture of monomers described above include peroxides such as lauroyl peroxide, cumene hydroperoxide, and benzoyl peroxide, which are general radical polymerization initiators, azobisvaleronitrile, and azobisisobutyronitrile. The addition amount of the polymerization initiator is preferably about 100 to 10,000 ppm with respect to the total amount of monomers.

When the crosslinked hydrophilic polymer (hydrogel) is selected as the material of the ring-shaped member 6 to be a part of the distal end portion 8 in the endoscope hood 1 according to the present embodiment, as described above, by setting the blending amount of the crosslinkable monomer to be blended in the polymerizable composition for forming the annular member 6 to be larger than the blending amount of the crosslinkable monomer to be blended in the polymerizable composition for forming the main body portion 5, the crosslinking density of the annular member 6 becomes higher than the crosslinking density of the main body portion 5, and it is possible to impart preferable strength to the distal end portion 8 when the annular member 6 (second distal end portion 7) and the main body portion 5 are combined to form the endoscope hood 1 according to the present embodiment.

The blending amount of the crosslinkable monomer to be used for forming the annular member 6 varies depending on the molecular weight of the crosslinkable monomer to be used, and in the case of a low molecular weight, the blending amount is preferably 5 wt% to 25 wt%, and more preferably 10 wt% to 20 wt% with respect to the total weight of the monomers that form the main body portion 5. In the case of a high molecular weight, the blending amount is preferably 25 wt% to 50 wt%, more preferably 30 wt% to 40 wt% based on the total weight of monomers that form the main body portion 5.

The endoscope hood 1 according to the present embodiment is formed into a desired shape when the above-described monomers are mixed singly or after a plurality of the above-described monomers are mixed. In the first aspect, the endoscope hood 1 is formed of a single material, but in the second aspect, the exterior portion 2 and the disk portion 3 can be formed of different materials and then used in combination. In this case, when the water content of the disk portion 3 is adjusted to be larger than the water content of the exterior portion 2, the antifogging properties and the antifouling properties are more effectively exhibited.

As a step of obtaining a polymer, a monomer mixed solution obtained by mixing monomers as constituent components is placed in a molding die such as a metal, glass, or plastic, sealed, heated stepwise or continuously in a thermostatic bath or the like in a range of 25 to 120°C, and the copolymerization reaction is completed in 5 to 120 hours, whereby a molding die containing a polymer can be obtained. For polymerization, ultraviolet rays, electron beams, gamma rays, and the like can be used.

As a step of obtaining a hydrogel, the molding die after completion of polymerization is cooled to room temperature, the polymer contained in the molding die is peeled off from the molding die, cutting and polishing are performed as necessary, and then the polymer is hydrated and swollen to form a hydrogel.

Examples of the liquid (swelling liquid) to be used include, but are not limited to, water, physiological saline, isotonic buffer, and a solution obtained by mixing an organic solvent such as ethanol with these. The swelling liquid is heated to 60 to 100°C, and the polymer is immersed in the swelling liquid for a certain period of time to be in a swollen state. In addition, it is preferable to remove unreacted monomers adhering to the polymer during the swelling treatment. The obtained hydrogel can be shaped by being subjected to high-pressure steam sterilization at 110 to 130°C for 10 to 60 minutes in a state of being immersed in a swelling liquid such as physiological saline.

### EXAMPLES

Hereinafter, the present invention will be described in more detail with reference to Examples, but the present invention is not limited to these Examples.

### (Method for Producing Endoscope Hood 1)

### <Example 1>

The annular member 6 of the endoscope hood 1 according to Example 1 is formed of an elastomer.

In Example 1, firstly, the annular member 6 made of silicone rubber molded in advance into a desired shape by injection molding was subjected to a corona discharge treatment, and then placed on the distal end portion 8 of a molding die for forming the endoscope hood 1 designed in advance to have the structure of the endoscope hood 1 shown in Table 1 after swelling.

Secondly, 99.0 g of 2-hydroxyethyl methacrylate as a hydrophilic monomer for forming the main body portion 5, 1.0 g of ethylene glycol dimethacrylate as a crosslinkable monomer, and 0.20 g of 2,2'-azobisisobutyronitrile as a polymerization initiator were mixed, then the mixture was dispensed into the above-described molding die on which the annular member 6 was placed, and subjected to thermal polymerization (heating to room temperature to 100°C under nitrogen atmosphere, 40 hours).

Third, the obtained copolymer was swollen by heating in physiological saline at 60°C for 30 minutes, and subjected to high-pressure steam sterilization to obtain the endoscope hood 1.

### <Example 2>

The annular member 6 of the endoscope hood 1 according to Example 2 is formed of a thermoplastic polymer.

In Example 2, firstly, the annular member made of polyvinyl chloride (PVC) annular member molded in advance into a desired shape by injection molding was subjected to a corona discharge treatment, and then placed on the distal end portion 8 of a molding die for forming the endoscope hood 1 designed in advance to have the structure of the endoscope hood 1 shown in Table 1 after swelling.

Secondly, 99.0 g of 2-hydroxyethyl methacrylate as a hydrophilic monomer for forming the main body portion 5, 1.0 g of ethylene glycol dimethacrylate as a crosslinkable monomer, and 0.30 g of 2,2'-azobisisobutyronitrile as a polymerization initiator were mixed, then the mixture was dispensed into the above-described molding die on which the annular member was placed, and subjected to thermal polymerization (heating to room temperature to 100°C under nitrogen atmosphere, 40 hours).

Third, the obtained copolymer was swollen by heating in physiological saline at 60°C for 30 minutes, and subjected to high-pressure steam sterilization to obtain the endoscope hood 1.

### <Example 3>

The annular member 6 of the endoscope hood 1 according to Example 3 is formed of a thermoplastic acrylic resin.

In Example 3, firstly, 90.0 g of methyl methacrylate as a monomer for forming the annular member 6, 10.0 g of PEG-200 dimethacrylate (4EG) as a crosslinkable monomer, and 0.30 g of 2,2'-azobisisobutyronitrile as a polymerization initiator were mixed, then the mixture was dispensed into a molding die for forming an endoscope hood, and subjected to thermal polymerization (heating to room temperature to 100°C under nitrogen atmosphere, 40 hours). The obtained annular member made of polymethyl methacrylate (PMMA) was subjected to a corona discharge treatment, and then placed on the distal end portion 8 of a molding die for forming the endoscope hood 1 designed in advance to have the structure of the endoscope hood 1 shown in Table 1 after swelling.

Second, 99.5 g of 2-hydroxyethyl methacrylate as a hydrophilic monomer for forming the main body portion 5, 0.5 g of ethylene glycol dimethacrylate as a crosslinkable monomer, and 0.30 g of 2,2'-azobisisobutyronitrile as a polymerization initiator were mixed, then the mixture was dispensed into the molding die in a state where the annular member was copolymerized, and newly subjected to thermal polymerization (heating to room temperature to 100°C under nitrogen atmosphere, 40 hours).

Third, the obtained copolymer was swollen by heating in physiological saline at 60°C for 30 minutes, and subjected to high-pressure steam sterilization to obtain an endoscope hood 13.

### <Example 4>

It is assumed that the annular member 6 of the endoscope hood 1 according to Example 4 is formed of a hydrogel of the same material as the main body portion 5.

In Example 3, firstly, 80.0 g of 2-hydroxyethyl methacrylate as a hydrophilic monomer for forming the annular member 6, 20.0 g of PEG-200 dimethacrylate (4EG) as a crosslinkable monomer, and 0.30 g of 2,2'-azobisisobutyronitrile as a polymerization initiator were mixed, and then the mixture was dispensed into a molding die for forming the endoscope hood 1 designed in advance to have the structure of the exterior portion 2 shown in Table 1 after swelling, and subjected to thermal polymerization (heating to room temperature to 100°C under nitrogen atmosphere, 40 hours).

Second, 96.5 g of 2-hydroxyethyl methacrylate as a hydrophilic monomer for forming the main body portion 5, 3.5 g of PEG-200 dimethacrylate (4EG) as a crosslinkable monomer, and 0.15 g of 2,2'-azobisisobutyronitrile as a polymerization initiator were mixed, then the mixture was dispensed into the molding die in a state where the annular member was copolymerized, and newly subjected to thermal polymerization (heating to room temperature to 100°C under nitrogen atmosphere, 40 hours).

Third, the obtained copolymer was swollen by heating in physiological saline at 60°C for 30 minutes, and subjected to high-pressure steam sterilization to obtain an endoscope hood 14.

### <Example 5>

It is assumed that the annular member 6 of the endoscope hood 1 according to Example 5 is formed of a hydrogel of a material different from that of the main body portion 5.

In Example 4, firstly, 70.0 g of 2-hydroxyethyl methacrylate as a hydrophilic monomer for forming the annular member 6, 30.0 g of trimethylolpropane triacrylate (TMPTA) as a crosslinkable monomer, and 0.30 g of 2,2'-azobisisobutyronitrile as a polymerization initiator were mixed, then the mixture was dispensed into a molding die for forming the endoscope hood 1 designed in advance to have the structure of the endoscope hood 1 shown in Table 1 after swelling, and subjected to thermal polymerization (heating to room temperature to 100°C under nitrogen atmosphere, 40 hours).

Secondly, 67.5 g of N,N-dimethyl (meth)acrylamide and 27.5 g of N-vinylpyrrolidone as hydrophilic monomers for forming the main body portion 5, 5.0 g of PEG-200 dimethacrylate (4EG) as a crosslinkable monomer, and 0.15 g of 2,2'-azobisisobutyronitrile as a polymerization initiator were mixed, then the mixture was dispensed into the molding die in a state where the annular member was copolymerized, and newly subjected to thermal polymerization (heating to room temperature to 100°C under nitrogen atmosphere, 40 hours).

Third, the obtained copolymer was swollen by heating in physiological saline at 60°C for 30 minutes, and subjected to high-pressure steam sterilization to obtain an endoscope hood 15.

### <Comparative Example 1>

It is assumed that the endoscope hood 1 according to Comparative Example 1 is not provided with the annular member 6.

In Comparative Example 1, firstly, 99.0 g of 2-hydroxyethyl methacrylate as a hydrophilic monomer for forming the main body portion 5, 1.0 g of ethylene glycol dimethacrylate as a crosslinkable monomer, and 0.30 g of 2,2'-azobisisobutyronitrile as a polymerization initiator were mixed, then the mixture was dispensed into the above-described molding die in a state where the annular member 6 was not present, and subjected to thermal polymerization (heating to room temperature to 100°C under nitrogen atmosphere, 40 hours).

Second, the obtained copolymer was swollen by heating in physiological saline at 60°C for 30 minutes, and subjected to high-pressure steam sterilization to obtain the endoscope hood 1.

### (Evaluation of Physical Properties)

Examples 1 to 5 and Comparative Example 1 were evaluated according to the following criteria.

### (Strength of Annular Portion)

The compressive stress was calculated according to the following for the annular member formed according to the above in Example 1 to 5, and for the annular portion 6 formed by the same method as in Example 3 in Comparative Example 1.

The annular portions 6 of Examples 1 to 5 and Comparative Example 1 formed in the shapes shown in Table 1 were set in a compression bending tester (manufactured by Shimadzu Corporation/model number EZ-SX50 N) such that the opening portion was on the near side, and then a load was started. The load amount at the time when 30% of the original outer diameter of the test piece (annular member) was compressed was measured and taken as the compressive stress.

### (Evaluation Method)

The compressive stress of each of 10 samples was measured, and the average value of 10 samples was calculated as 30% deformation compressive bending strength (N) of Examples 1 to 5 and Comparative Example 1, and evaluated according to the following criteria.
○: Compressive stress is 1.0 N to 2.0 N.
Δ: The compressive stress is less than 1.0 N, or the compressive stress is more than 2.0 N.
×: Measurement is not possible.

The method of evaluating the elasticity of the distal end portion is as follows.

**[Table 1]**

| | Annual Portion | Main Body Portion | Entire Shape | 30% Deformation Compressive Bending Strength (N) of Annular Portion | Evaluation Result |
|---|---|---|---|---|---|
| Example 1 | Material: Thermoplastic elastomer (silicone rubber) | Material: Thermosetting polymer (wt%) (HEMA99.0, ED1.0) | Length of distal end portion 5.0(mm) / Thickness of distal end portion 0.5(mm) | 1.0995 | ○ |
| | Inner diameter: 11.0(mm) | Inner diameter: 11.5(mm) | Thickness of disk portion 0.5(mm) | | |
| | Outer diameter: 11.5(mm) | Outer diameter: 12.0(mm) | Length of exterior portion 12.0(mm) / Thickness of exterior portion 0.5(mm) | | |
| Example 2 | Material: Thermoplastic polymer (polyvinyl chloride) | Material: Thermosetting polymer (wt%) (HEMA99.0, ED1.0) | Length of distal end portion 3.5(mm) / Thickness of distal end portion 0.5(mm) | 1.5560 | ○ |
| | Inner diameter: 10.0(mm) | Inner diameter: 11.5(mm) | Thickness of disk portion 0.5(mm) | | |
| | Outer diameter: 11.0(mm) | Outer diameter: 12.0(mm) | Length of exterior portion 12.5(mm) / Thickness of exterior portion 0.5(mm) | | |
| Example 3 | Material: Thermosetting polymer (wt%) (MMA90, 4EG10) | Material: Thermosetting polymer (wt%) (HEMA99.5, ED0.5) | Length of distal end portion 3.5(mm) / Thickness of distal end portion 0.5(mm) | 1.5976 | ○ |
| | Inner diameter: 10. (mm) | Inner diameter: 11.5(mm) | Thickness of disk portion 0.5(mm) | | |
| | Outer diameter: 11.5(mm) | Outer diameter: 12.0(mm) | Length of exterior portion 12.0(mm) / Thickness of exterior portion 0.5(mm) | | |
| Example 4 | Material: Thermosetting polymer (wt%) (HEMA80, 4EG20) | Material: Thermosetting polymer (wt%) (HEMA96.5, 4EG3.5) | Length of distal end portion 3.0(mm) / Thickness of distal end portion 0.3(mm) | 1.5945 | ○ |
| | Inner diameter: 9.7(mm) | Inner diameter: 9.7(mm) | Thickness of disk portion 0.5(mm) | | |
| | Outer diameter: 10.0(mm) | Outer diameter: 10.0(mm) | Length of exterior portion 10.0(mm) / Thickness of exterior portion 0.3(mm) | | |
| Example 5 | Material: Thermosetting polymer (wt%) (HEMA70, TMPTA30) | Material: Thermosetting polymer (wt%) (DMAA67.5. NVP27.5, 4EG5.0) | Length of distal end portion 4.5(mm) / Thickness of distal end portion 0.5(mm) | 1.8562 | ○ |
| | Inner diameter: 11.5(mm) | Inner diameter: 11.5(mm) | Thickness of disk portion 0.3(mm) | | |
| | Outer diameter: 12.0(mm) | Outer diameter: 12.0(mm) | Length of exterior portion 12.5(mm) / Thickness of exterior portion 0.5(mm) | | |
| Comparative Example 1 | Material: Thermosetting polymer (wt%) (HEMA99.0, ED1.0) | Material: Thermosetting polymer (wt%) (HEMA99.0, ED1.00) | Length of distal end portion 4.5(mm) / Thickness of distal end portion 0.5(mm) | Measurement is not possible (not standing alone) | × |
| | Inner diameter: 10.5(mm) | Inner diameter: 11.5(mm) | Thickness of disk portion 0.3(mm) | | |
| | Outer diameter: 11.5(mm) | Outer diameter: 12.0(mm) | Length of exterior portion 12.5(mm) / Thickness of exterior portion 0.5(mm) | | |

Since the endoscope hood 1 according to the present embodiment is excellent in antifogging properties and antifouling properties, it is possible to easily remove body fluid and oil adhering to the lens part of the endoscope with water ejected from the water supply mechanism provided for the endoscope at the time of surgery or examination, and further, it is possible to suppress fogging.

In addition, according to the endoscope hood 1 according to the present embodiment, since the distal end portion has sufficient strength and elasticity to push apart the biological tissue, operability when using the endoscope is improved.

As described above, the present invention has been described with reference to the embodiments, but it should be understood that the present invention is not limited to the description and drawings constituting a part of the disclosure in such embodiments. Various alternative embodiments, examples, and operations will be apparent to those skilled in the art from such disclosure.

### REFERENCE SIGNS LIST

- 1: endoscope hood
- 2: exterior portion
- 3: disk portion
- 4: first distal end portion
- 5: main body portion
- 6: annular member
- 7: second distal end portion
- 8: distal end portion
- 9: forceps port
- 10: water supply port
- a: length of distal end portion
- b: length of exterior portion
- c: thickness of exterior portion
- d: thickness of disk portion
- e: inner diameter of exterior portion
- e2: inner diameter of annular member
- f: outer diameter of exterior portion
- f2: outer diameter of annular member
- g: thickness of distal end portion

## Claims

1. An endoscope hood comprising:
a main body portion made of a hydrogel; and
a second distal end portion, wherein
the main body portion includes:
an exterior portion having a substantially cylindrical shape,
a disk portion circumferentially in contact with an inner surface of the exterior portion, and
a first distal end portion connected to an insertion-side end portion of the exterior portion, and
the second distal end portion is connected to an insertion-side end portion of the first distal end portion and includes an annular member.

2. The endoscope hood according to claim 1, wherein
30% deformation compressive bending strength of the annular member is stronger than 30% deformation compressive bending strength of the main body portion.

3. The endoscope hood according to claim 1, wherein
the annular portion is selected from thermoplastic elastomers selected from styrene-based, olefin-based, PVC-based, EVA-based, urethane-based, and acryl-based, and thermosetting elastomers selected from vulcanized rubber and resin-based elastomers.

4. The endoscope hood according to claim 1, wherein
the annular portion is selected from thermosetting polymers having elasticity.

5. The endoscope hood according to any one of claims 1 to 4, wherein
the 30% deformation compressive bending strength of the annular portion is 1.0 N to 2.0 N.
